# EUROPEAN PATENT SPECIFICATION

(11) **EP 3 941 926 B1**
(45) Date of publication and mention of the grant of the patent: **26.04.2023**
(21) Application number: 20708504.4
(22) Date of filing: 11.03.2020
(51) Int. Cl.: C07J 51/00

(54) **PROCESS FOR THE PREPARATION OF FULVESTRANT 3-BORONIC ACID**
VERFAHREN ZUR HERSTELLUNG VON FULVESTRANT-3-BORSÄURE
PROCÉDÉ DE PRÉPARATION D'ACIDE FULVESTRANT 3-BORONIQUE

(30) Priority: 20.03.2019 IT 201900004041
(43) Date of publication of application: 26.01.2022
(73) Proprietor: Farmabios S.p.A., 27027 Gropello Cairoli (Pavia) (IT)
(72) Inventor: GABOARDI, Mauro, 27027 Gropello Cairolo PV (IT); MANFROTTO, Cristina, 27027 Gropello Cairoli PV (IT); DI GIACOMO, Mario, 27027 Gropello Cairoli PV (IT)
(74) Representative: Longoni, Alessandra
(86) International application number: PCT/EP2020/056463
(87) International publication number: WO 2020/187658

(56) References cited:
- WO-A1-2008/065100
- WO-A1-2016/004166
- JIAWANG LIU ET AL: "Fulvestrant-3 Boronic Acid (ZB716): An Orally Bioavailable Selective Estrogen Receptor Downregulator (SERD)", JOURNAL OF MEDICINAL CHEMISTRY, vol. 59, no. 17, 29 August 2016 (2016-08-29), pages 8134-8140, XP055645152, US ISSN: 0022-2623, DOI: 10.1021/acs.jmedchem.6b00753 cited in the application

## Description

The present invention relates to a process for the preparation of a fulvestrant derivative, in particular fulvestrant-3 boronic acid, and intermediates useful for its preparation.

### Background of the Invention

Fulvestrant is a selective estrogen receptor downregulator (SERD) able to competitively and reversibly bind to said receptor, resulting in its downregulation and degradation.

Fulvestrant has been first approved as a drug in the USA in 2002 and then in Europe in 2004 under the tradename Faslodex^{®}. It is indicated as monotherapy for the treatment of estrogen receptor positive, locally advanced or metastatic breast cancer in postmenopausal women not previously treated with endocrine therapy or with disease relapse on antiestrogen therapy and, in combination with palbociclib, for the treatment of hormone receptor (HR)-positive, human epidermal growth factor receptor 2 (HER2)-negative locally advanced or metastatic breast cancer in women who have received prior endocrine therapy.

It is known that fulvestrant, when orally administered, is subject to fast O-glucuronidation and O-sulfation to give phase II polar metabolites which are inactive and water-soluble. The metabolic inactivation and high clearance make fulvestrant poorly or not available to the target tissues.

Therefore, due to its poor oral bioavailability, fulvestrant must be administered by intramuscular injection.

Rather recently, a fulvestrant derivative, fulvestrant-3 boronic acid, has been developed wherein the hydroxy group in 3 has been replaced by a boronic acid group in order to prevent the early metabolic inactivation. Such a change, while maintaining unchanged the steroid moiety which confers its SERD properties, reduces the metabolic inactivation of fulvestrant thereby making it bioavailable also after oral administration (Jiawang Liu et al., "Fulvestrant-3 Boronic Acid (ZB716): An Orally Bioavailable Selective Estrogen Receptor Downregulator (SERD)" J. Med. Chem. 2016, 59, 8134-8140).

Fulvestrant-3 boronic acid, CAS chemical name ((7R,8R,9S,13S,14S,17S)-17-hydroxy-13-methyl-7 -(9-((4,4,5,5,5-pentafluoropentyl)sulfinyl)nonyl)-7,8,9,11,12,13,14,15,16,17-decahydro-6H-cyclopenta[a]phenanthren-3-yl)boronic acid, is the compound of formula (I): 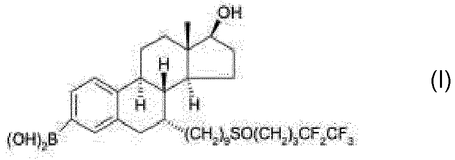 first described in the International patent application WO2016004166.

All the processes for the preparation of fulvestrant-3 boronic acid which are known in the art provide for the use of intermediates which are difficult to handle and to purify and result in several disadvantages.

In particular, in the literature (J. Med. Chem. 2016, 59, 8134-8140) it is known a process for the preparation of fulvestrant-3 boronic acid (Scheme I) which comprises the esterification of 17-acetyl S-deoxo fulvestrant with triflic anhydride to give the corresponding triflate (2), which is then reacted with bis(pinacolato)diboron in the presence of palladium(II) acetate and tricyclohexylfosfine to give the 3-pinacolyl boronate ester (3). After removal of the 17-acetyl group under basic conditions, the deacetyl boronate ester (4) is oxidized with *meta*-chloroperoxybenzoic acid (mCPBA) to give the final product as colourless crystals.

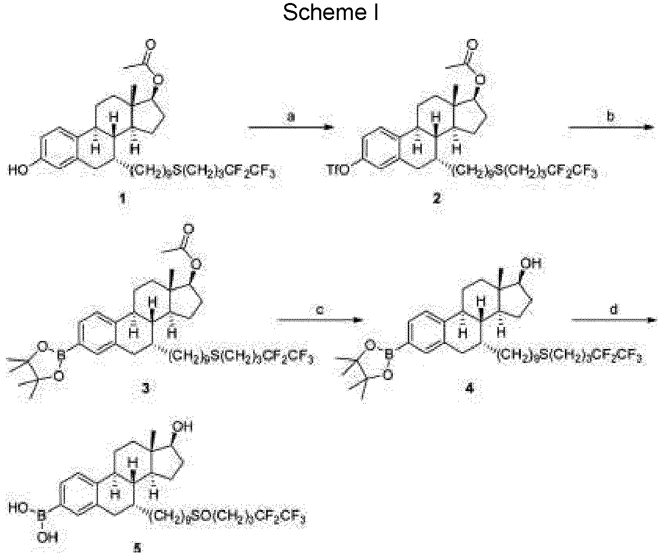

However, since the boronate esters (3 and 4) are oils, they are difficult to isolate and require complex purification steps which decrease the overall yield and the purity of the final product. Moreover, the ester 4 is unstable under chromatographic conditions and results in the formation of variable amounts of other undesired products, with consequent loss of yield.

Therefore, there is still the need for an improved process for the synthesis of fulvestrant-3 boronic acid which overcomes the drawbacks of the known processes.

### Summary of the Invention

Object of the present invention is a process for the preparation of fulvestrant-3 boronic acid which occurs through the formation of potassium 17-acetyl S-deoxo fulvestrant 3-trifluoroborate (in short potassium fulvestrant 3-trifluoroborate). The compound potassium fulvestrant 3-trifluoroborate is a further object of the present invention.

### Brief Description of the Drawings

Figure 1: XRPD spectrum of potassium fulvestrant 3-trifluoroborate.

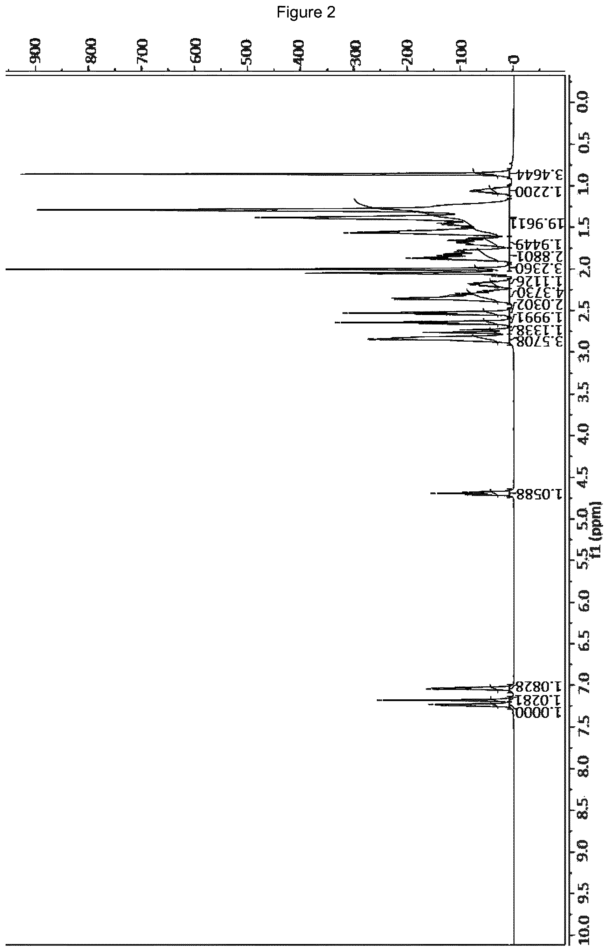

Figure 2: ¹H NMR spectrum of potassium fulvestrant 3-trifluoroborate.

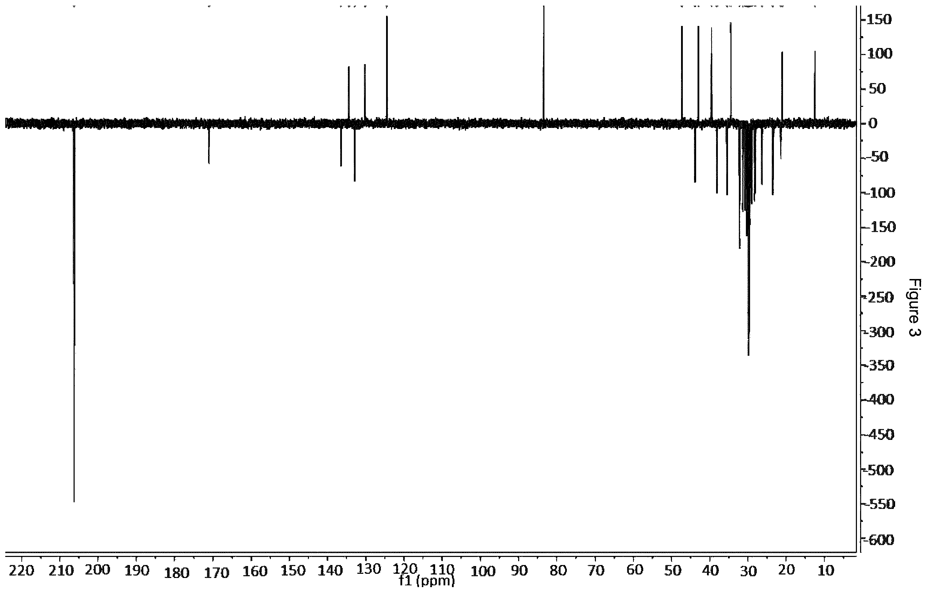

Figure 3: ¹³C NMR spectrum of potassium fulvestrant 3-trifluoroborate.

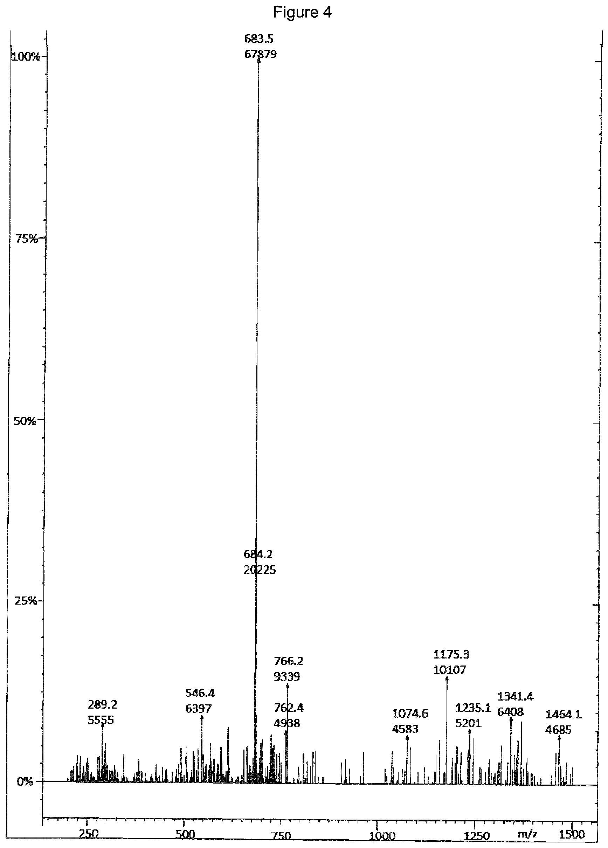

Figure 4: LC-MS spectrum of potassium fulvestrant 3-trifluoroborate.
Figure 5: Chromatogram of potassium fulvestrant 3-trifluoroborate prior to re-crystallization.
Figure 6: Chromatogram of potassium fulvestrant 3-trifluoroborate after re-crystallization.

### Detailed Description of the Invention

After extensive experimentation, the inventors of the present invention have now surprisingly found that, starting from fulvestrant 3-pinacolyl boronate obtained for example following the process described in WO 2016/004166, a new intermediate, potassium fulvestrant 3-trifluoroborate, can be obtained and advantageously used in the synthesis of fulvestrant-3 boronic acid.

Therefore, object of the present invention is a process for the synthesis of fulvestrant-3 boronic acid comprising:
the transformation of a compound of formula (IV) 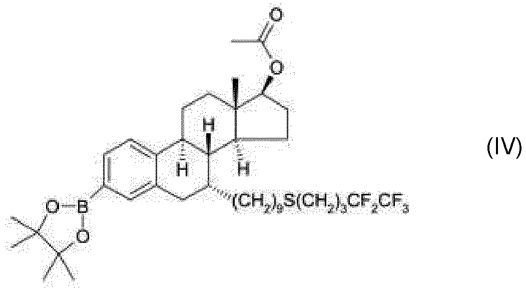 into a compound of formula (III)

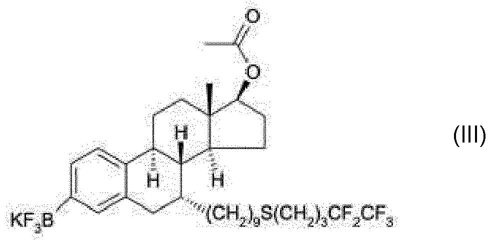

In an embodiment, said transformation provides for the reaction of 17-acetyl S-deoxo fulvestrant 3-pinacolyl boronate (IV) with potassium hydrogen difluoride (KHF₂), in a suitable reaction solvent to give potassium fulvestrant 3-trifluoroborate (III).

The molar ratio between the compound of formula (IV) and potassium hydrogen difluoride can be from about 3 to about 8, preferably 7.

Potassium hydrogen difluoride can be used as an aqueous solution having a concentration from 2 M to 5 M, preferably 4.5 M.

According to the present invention, a suitable reaction solvent can be any water-miscible solvents. For example, the solvent can be selected among: acetonitrile, tetrahydrofuran (THF), methanol, acetone, water and mixtures thereof. In a particularly preferred embodiment of the present invention, the solvent is a mixture of water and acetone.

Said transformation can be carried out by keeping the reaction mixture under stirring at a temperature from 15 to 30°C, preferably at room temperature, for a time period of about 60 - 120 minutes, preferably for 90 minutes. Anyway, the temperature as well as longer reaction times are not a critical parameter of the present transformation.

The compound of formula (III), obtained from the above transformation, is a further object of the present invention. Surprisingly, the compound of formula (III), differing from the intermediates for the synthesis of fulvestrant 3-boronic acid known in the art, is a crystalline solid. Said crystalline solid can be obtained by adding a mixture of a suitable hydrocarbon and water (crystallization mixture) to the mixture of reaction end to obtain the precipitation of the compound of formula (III).

The volume ratio between hydrocarbon and water in the crystallization mixture can be between about 5:1 and about 5:2, preferably 16:5.

Suitable hydrocarbons can be selected, for example, among: heptane, hexane, pentane and methyl-cyclohexane. In a preferred embodiment, the mixture is a mixture of heptane and water.

In a particularly preferred embodiment, the crystallization of the compound of formula (III) occurs as reported herein after: a crystallization mixture heptane:water 16:5 is added to the reaction mixture, the crude solid precipitate is filtered, washed with a mixture heptane:water 1:1 and then dried at a temperature from 30°C to 50°C, preferably at about 40°C, for a suitable period of time, for example, from 2 to 10 hours, preferably about 5 hours. The drying can be carried out according to methods known in the art including, but not limited to, vacuum oven, Rotavapor^{®}, air-drying chamber, static bed dryer, fluid bed dryer, spray dryer and the like. Preferably, the drying is carried out by drying in oven under vacuo at 40°±5°C.

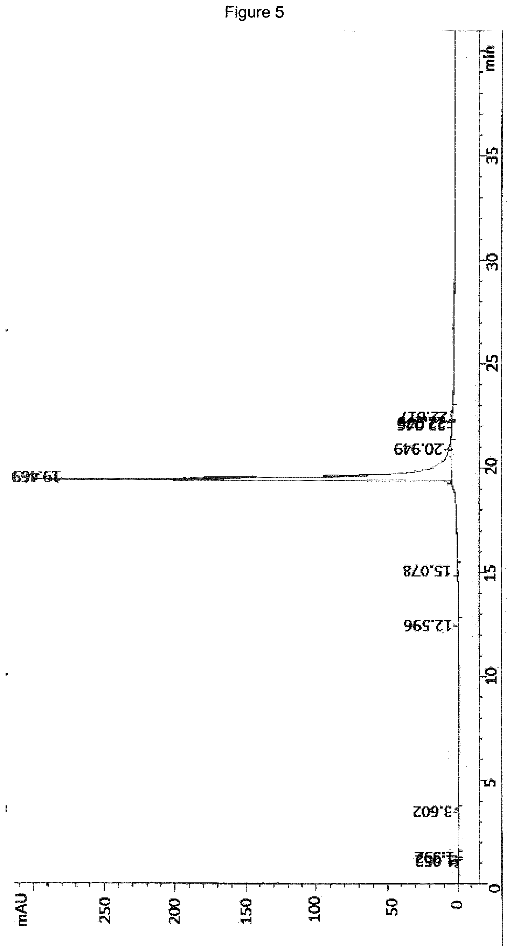

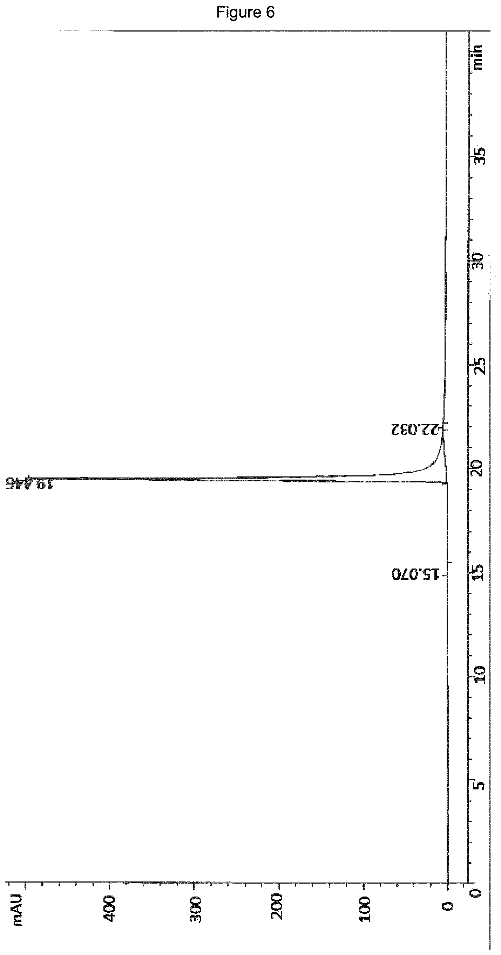

This intermediate (III) is obtained with already a very good quality, that is with a purity > 97.0% and single impurities not more than 1.0% (Figure 5). Moreover, the optional re-crystallization results in a product with purity > 99.8% in high yield (recover > 90%) (Figure 6).

Said re-crystallization can be carried out according to methods known in the art, in particular, by hot-cold crystallization wherein first potassium fulvestrant 3-trifluoroborate is dissolved at warm in a suitable solvent such as, for example, acetonitrile, then the resulting solution is cooled to obtain the precipitation of the product and finally the precipitate is separated by filtration and dried.

It is worth noting that, in addition to avoid the drawback of oily intermediates, the compound of formula (III) is obtained in crystalline form and it is stable throughout all the purification steps.

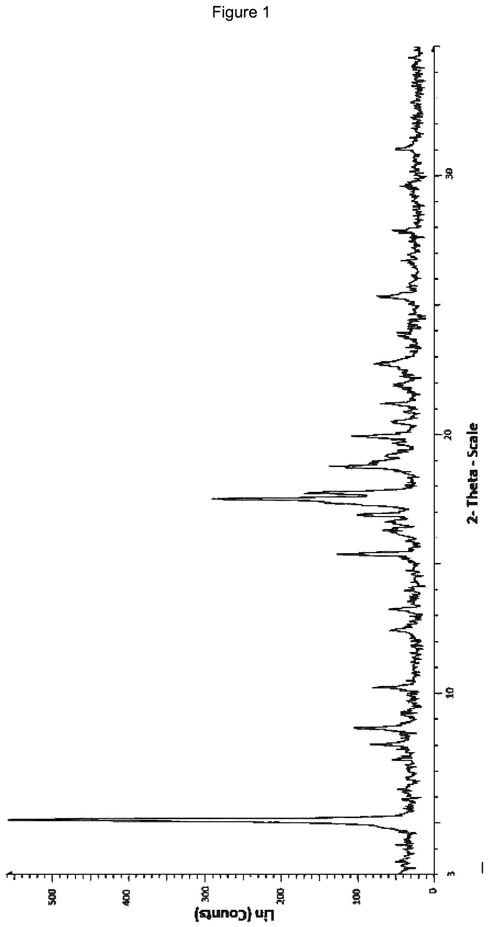

The compound of formula (III), obtained according to the present invention, has been characterized also by X-ray powder diffractometry (XRPD) and shows, in the XRPD diffractogram (Figure 1), at least three of the following characteristic peaks: 5.09, 8.03, 8.61, 10.20, 15.37, 17.72 ± 0.2 degrees 2θ.

Fulvestrant-3 boronic acid can be obtained from the compound of formula (III) according to conventional methods.

However, the inventors have also developed processes for the preparation of fulvestrant-3 boronic acid which occur through the formation of the intermediate compound of formula (III).

According to the present invention, the compound of formula (III), potassium 17-acetyl S-deoxo fulvestrant 3-trifluoroborate, can undergo an oxidation reaction on the sulfur atom and a hydrolysis reaction of the acetyl group in position 17 and the potassium trifluoroborate group in position 3, or viceversa, to give fulvestrant 3-boronic acid.

Therefore, a particularly preferred embodiment of the present invention is a process for the preparation of fulvestrant-3 boronic acid further comprising the following steps:
- oxidation reaction of the compound of formula (III) to give a compound of formula (IIa) 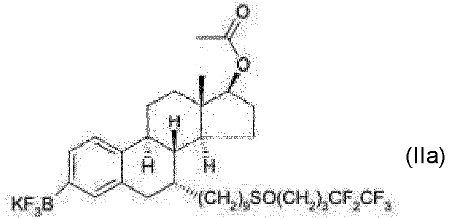 and
- hydrolysis reaction of the compound of formula (Ila) to give fulvestrant 3-boronic acid (I).

Another particularly preferred embodiment of the present invention is a process for the preparation of fulvestrant-3 boronic acid further comprising the following steps:
- hydrolysis reaction of the compound of formula (III) to give the compound of formula (IIb) 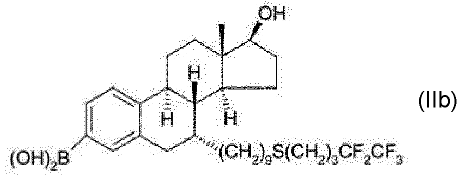 and
- oxidation reaction of the compound of formula (IIb) to give fulvestrant 3-boronic acid (I).

More particularly, the hydrolysis reaction can be carried out by reacting the compound of formula (IIa) or (III), respectively, with a base in a suitable reaction solvent.

A suitable base can be an alkaline metal hydroxide selected among lithium hydroxide, potassium hydroxide and sodium hydroxide, preferably lithium hydroxide. The oxidation reaction can be carried out by reacting the compound of formula (III) or (IIb), respectively, with a suitable oxidizing agent in a suitable reaction solvent.

A suitable oxidazing agent can be selected, for example, between sodium periodate and metachloroperbenzoic acid, preferably sodium periodate.

A suitable reaction solvent can be a protic or aprotic polar solvent selected, for example, among methanol, acetonitrile, acetone, tetrahydrofuran, water, dimethylformamide, dimethylacetamide and mixtures thereof. Preferably, in the oxidation reaction, the suitable solvent is a mixture of water, tetrahydrofuran and methanol.

Even if it is not strictly necessary, preferably the above oxidation and hydrolysis reactions are carried out under inert atmosphere, for example, under nitrogen atmosphere.

Moreover, it is worth noting that both intermediate compounds of formula (IIa) and (IIb) are solid.

Advantageously, fulvestrant-3 boronic acid obtained according to the present invention has a purity even higher than 95% without requiring any chromatographic column; by using one chromatography a purity higher than 99% can be achieved. Without being bound to any theory, the inventors of the present invention believe that this could be due just to the use of the intermediate compound of formula (III) which allows to avoid the use of oily intermediates difficult to purify by traditional methods including no chromatography.

Therefore, notwithstanding the invention has been described in details, the only essential feature of the present process for the preparation of fulvestrant-3 boronic acid is that it occurs through the formation of the intermediate compound of formula (III), fulvestrant-3 potassium trifluoroborate.

All terms as used in the present disclosure, unless otherwise indicated, should be understood in their common meaning as known in the field.

The term "about" includes the range of experimental error which can occur in a measurement. In particular, when referred to a value, it means given value plus or minus 5% and, when referred to a range, it means the outer values plus or minus 5%.

Herein after, the present invention will be described by means of some examples which have an illustrative purpose only and should not be considered as limiting the scope of the invention, which is defined in the appended claims.

### EXAMPLES

The X ray diffraction spectra (XRPD) of the compound of formula (III) have been performed with a Bruker D5005 diffractometer equipped with CuKα radiation, a scintillation detector and a curved graphite monochromator on the diffracted beam. The samples of the compound of formula (III) after crystallization from acetonitrile were mildly grinded in an agate mortar to obtain a fine powder and disintegrate any particle agglomerates. Data have been collected at room temperature in a silicon monocrystalline low-background sample holder. Detection: 2θ degree, measurement of the angular range from 3° to 35° (2θ), with a step of 0.03° and counting time of 4 s/step.

The HPLC chromatograms have been performed by using an Agilent 1200 Series equipment by injecting 10 µL solution in a RP18 column; 150 x 4.6 mm; 5 µm. The sample was eluted in gradient with a mobile phase consisting of a mixture of acetonitrile and water with the addition of phosphoric acid. The compounds were then analysed by applying a wavelength of 225 nm.

The ¹H and ¹³C NMR spectra were obtained by a Bruker AVANCE III spectrometer (500 MHz) at 25°C observing ¹H and ¹³C at 500 and 125.8 MHz, respectively. The chemical shifts are expressed in ppm with respect to tetramethylsilane and the spectra were obtained by dissolving the sample in acetone.

The LC-mass analysis were carried out by using a Varian 500 MS equipment in ESI(-).

### EXAMPLE 1: Synthesis of potassium ((7R,8R,9S,13S,14S,17S)-17-acetoxy-13-methyl-7-(9-((4,4,5,5,5-pentafluoropentyl)thio)nonyl)-7,8,9,11,12,13,14,15,16,17-decahydro-6H-cyclopenta[a]phenanthren-3-yl)trifluoroborate (III).

32 g of a crude residue obtained as described in the literature (WO2016004166 - Example 2, step 2) containing (7R,8R,9S,13S,14S,17S)-13-methyl-7-(9-((4,4,5,5,5-pentafluoropentyl)thio)nonyl)-3-(4,4,5,5-tetramethyl-1,3,2-dioxaborolan-2-yl)-7,8,9,11,12,13,14,15,16,17-decahydro-6H-cyclopenta[a]phenanthren-17-yl acetate was dissolved in acetone (85.5 ml) and, under stirring, demineralized water (38,5 ml) and an aqueous 4.5 M potassium hydrogen difluoride solution (57 ml) were added. The reaction mixture was kept at room temperature for 90 minutes. At the end of the reaction, water (160 ml) and heptane (512 ml) were added. The solid was filtered and washed with a mixture of heptane (70 ml) and water (70 ml). The solid was dried in oven under vacuum at 40°C for 5h. 24 g of potassium ((7R,8R,9S, 13S, 14S, 17S)-17-acetoxy-13-methyl-7-(9-((4,4,5,5,5-pentafluoropentyl)-thio)nonyl)-7,8,9,11,12,13,14,15,16,17-decahydro-6H-cyclopenta[a]phenanthren-3-yl)trifluoroborate (III) were obtained with HPLC purity 97.2%.

Furthermore, the compound of formula (III) can be re-crystallized. 24 g of the compound of formula (III) were dissolved in 60 ml acetonitrile at the reflux temperature and subsequently cooled to 0-10°C. The product was filtered and washed with 24 ml acetonitrile at 0-10°C. The product was dried for 5 h under vacuum at 40°C. 22 g of compound of formula (III) with HPLC purity >99.8% were obtained.

¹H NMR: 7.24 (1H, d, H-1); 7.18 (1H, s, H-4); 7.04 (3H, d, H-4); 4.69 (1H, *t*, H-17); 2.65 (2H, *t,* -CH₂-S); 2.53 (2H, *t,* -CH₂-S); 2.00 (3H, s, -CH₃); 0.86 (3H, *s*, -CH₃).

¹³C NMR: 12.5 (CH₃); 21.0 (CH₃); 21.4 (tCH₂); 29.9 (tCH₂); 32.2-23.5 (nCH₂); 34.5 (CH); 35.5 (CH₂); 38.1 (CH₂); 39.6 (CH); 43.0 (CH); 43.9 (C); 47.2; 83.3, 124.5, 130.2 (CH); 133.0 (C); 134.4 (CH); 136.5, 171.0, 206.3 (C).

### EXAMPLE 2: Synthesis of potassium ((7R,8R,9S,13S,14S,17S)-17-acetoxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl)trifluoroborate (Ila)

In a round flask and under nitrogen, the compound of formula (III) (5.0 g, 6.9 mmol) was dissolved in THF (50 ml) and methanol (10 ml) under stirring and the solution was brought to 0-5°C. In another round flask a solution of sodium periodate (2.95 g, 13.8 mmol) in water (12 ml) at 30-35°C was prepared. The solution of sodium periodate was added at 0-5°C to the reaction mixture. Subsequently the temperature was brought to 20-25°C and the reaction mixture was kept under stirring for 24-48 h until the completion of the reaction. The resultant solid was filtered, washed with THF and the oxidizing strength was destroyed with a 10% w/w aqueous solution of sodium thiosulfate (10 ml). The organic solvents were removed under vacuum and the mixture was taken up in ethyl acetate (50 ml). The phases were separated and the aqueous phase was extracted again with ethyl acetate (25 ml). The collected organic phases were washed once with water (25 ml) and once with a saturated sodium chloride solution (25 ml). The solvent was evaporated to residue to give 4.5 potassium ((7R,8R,9S,13S,14S,17S)-17-acetoxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl)trifluoroborate (IIa) with HPLC purity 96.5%.

### EXAMPLE 3: Synthesis of ((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl)boronic acid (I)

In a round flask and under nitrogen the compound of formula (IIa) (3.0 g, 4.0 mmol) was dissolved in acetonitrile (30 ml) and water (10 ml). Separately, a solution of lithium hydroxide (1.2 g, 28.0 mmol) in water (10 ml) was prepared and added, under stirring, to the reaction mixture at 0-5°C. The temperature was brought to 20-25°C and the mixture was allowed to react for 24-48 h. When the reaction was completed, a solution of ammonium chloride and hydrochloric acid up to pH 5-6 and ethyl acetate (30 ml) were added. The phases were separated and the aqueous phase was extracted again with ethyl acetate (15 ml). The collected organic phases were washed once with water (15 ml) and once with a saturated sodium chloride solution (15 ml). The solvent was evaporated to residue to give 2.0 g ((7R,8R,9S, 13S, 14S, 17S)-17-hydroxy-13-methyl-7-(9-((4,4,5,5,5-pentafluoropentyl)-sulfinyl)nonyl)-7,8,9,11,12,13,14,15,16,17-decahydro-6H-cyclopenta[a]phenanthren-3-yl)boronic acid (I) with HPLC purity 95.0%.

### EXAMPLE 4: Synthesis of ((7R,8R,9S,13S,14S, 17S)-17-hydroxy-13-methyl-7-(9-((4,4,5,5,5-pentafluoropentyl)thio)nonyl)-7,8,9,11,12,13,14,15,16,17-decahydro-6H-cyclopenta[a]phenanthren-3-yl)boronic acid (IIb)

In a round flask and under nitrogen the compound of formula (III) (1.0 g, 1.38 mmol) was dissolved in THF (5 ml) and MeOH (5 ml). Separately, a solution of potassium hydroxide (0.78 g, 13.8 mmol) in methanol (5 ml) was prepared and added, under stirring, to the reaction mixture at 0-5°C. The temperature was brought to 20-25°C and the mixture was allowed to react for 18 h. When the reaction was completed, acetic acid was added up to pH 5-6. The organic solvents were removed under vacuum and ethyl acetate (10 ml) was added. The phases were separated and the aqueous phase was extracted again with ethyl acetate (5 ml). The collected organic phases were washed once with water (5 ml) and once with a saturated sodium chloride solution (5 ml). The solvent was evaporated to residue to give 0.85 g ((7R,8R,9S,13S,14S,17S)-17-hydroxy-13-methyl-7-(9-((4,4,5,5,5-pentafluoropentyl)-thio)nonyl)-7,8,9,11,12,13,14,15,16,17-decahydro-6H-cyclopenta[a]phenanthren-3-yl)boronic acid (IIb) with HPLC purity 94.2%.

### EXAMPLE 5: Synthesis of ((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl)boronic acid (I)

In a round flask and under nitrogen the compound of formula (IIb) (0.66 g, 1.07 mmol) was dissolved in THF (7 ml) and methanol (1.2 ml) under stirring. In another round flask a solution of sodium periodate (0.39 g, 1.7 mmol) in water (2 ml) at 30-35°C was prepared. The solution of sodium periodate was added at 0-5°C to the reaction mixture. Subsequently the temperature was brought to 20-25°C and the reaction mixture was kept under stirring for 24-48 h until the completion of the reaction. The resultant solid was filtered, washed with THF and the oxidizing strength was destroyed with a 10% w/w aqueous solution of sodium thiosulfate (5 ml). The organic solvents were removed under vacuum and the mixture was taken up in ethyl acetate (10 ml). The phases were separated and the aqueous phase was extracted again with ethyl acetate (5 ml). The collected organic phases were washed once with water (5 ml) and once with a saturated sodium chloride solution (25 ml). The solvent was evaporated to residue to give 0.6 g ((7R,8R,9S,13S,14S,17S)-17-hydroxy-13-methyl-7-(9-((4,4,5,5,5-pentafluoropentyl)-sulfinyl)nonyl)-7,8,9,11,12,13,14,15,16,17-decahydro-6H-cyclopenta[a]phenanthren-3-yl)boronic acid (I). HPLC purity 92%.

## Claims

1. A process for the preparation of fulvestrant 3-boronic acid (I) comprising the transformation of a compound of formula (IV) 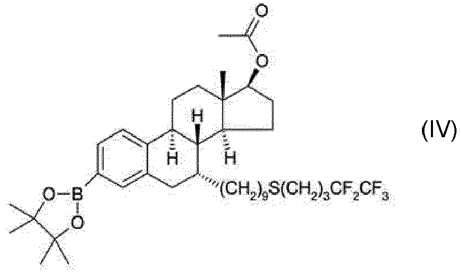 into a compound of formula (III)

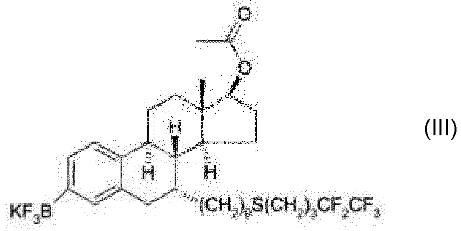

2. The process according to claim 1, wherein said transformation occurs by the reaction of the compound of formula (IV) with potassium hydrogen difluoride in a suitable reaction solvent.

3. The process according to claim 2, wherein the solvent is a solvent miscible with water preferably selected between acetonitrile, tetrahydrofuran, methanol, acetone, water and mixtures thereof.

4. The process according to claim 3, wherein the solvent is a mixture of water and acetone.

5. The process according to claim 1 further comprising the following steps:
- oxidation reaction of the compound of formula (III) to give a compound of formula (IIa) 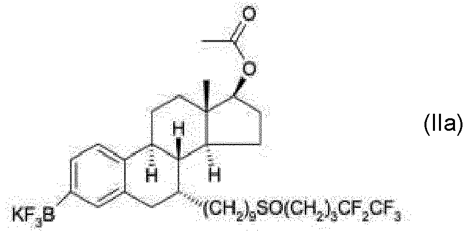 and
- hydrolysis reaction of the compound of formula (Ila) to give fulvestrant 3-boronic acid (I).

6. The process according to claim 1 further comprising the following steps:
- hydrolysis reaction of the compound of formula (III) to give the compound of formula (Ilb) 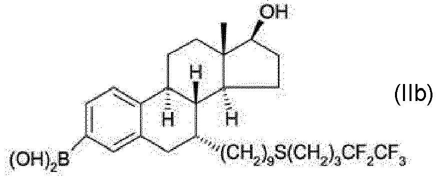 and
- oxidation reaction of the compound of formula (IIb) to give fulvestrant 3-boronic acid (I).

7. A compound of formula (III), as defined in claim 1.

8. The compound according to claim 7 having in the XRPD diffractogram at least three of the following characterising peaks: 5.09, 8.03, 8.61, 10.20, 15.37, 17.72 ± 0.2 degrees 2θ.

## Patentansprüche

1. Verfahren zur Herstellung von Fulvestrant-3-Boronsäure (I), umfassend die Umwandlung einer Verbindung der Formel (IV) 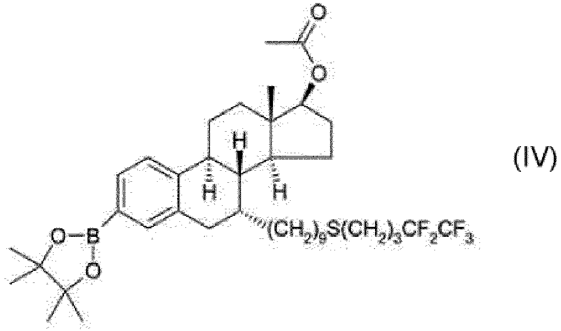 in eine Verbindung der Formel (III)

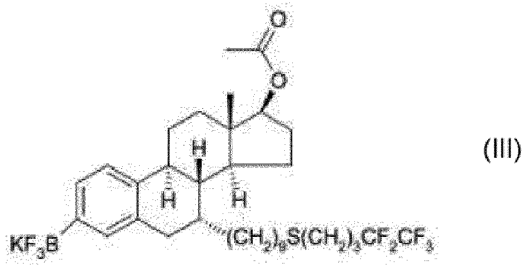

2. Verfahren nach Anspruch 1, wobei die Umwandlung durch die Reaktion der Verbindung der Formel (IV) mit Kaliumhydrogendifluorid in einem geeigneten Reaktionslösungsmittel erfolgt.

3. Verfahren nach Anspruch 2, wobei das Lösungsmittel ein mit Wasser mischbares Lösungsmittel ist, das bevorzugt aus Acetonitril, Tetrahydrofuran, Methanol, Aceton, Wasser und Mischungen davon ausgewählt ist.

4. Verfahren nach Anspruch 3, wobei das Lösungsmittel eine Mischung aus Wasser und Aceton ist.

5. Verfahren nach Anspruch 1, ferner die folgenden Schritte umfassend:
- Oxidationsreaktion der Verbindung der Formel (III) zum Bilden einer Verbindung der Formel (IIa) 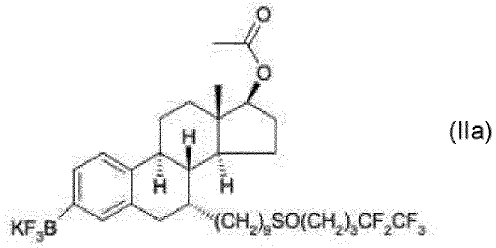 und
- Hydrolysereaktion der Verbindung der Formel (IIa) zum Bilden von Fulvestrant-3-Boronsäure (I).

6. Verfahren nach Anspruch 1, ferner die folgenden Schritte umfassend:
- Hydrolysereaktion der Verbindung der Formel (III) zum Bilden der Verbindung der Formel (IIb) 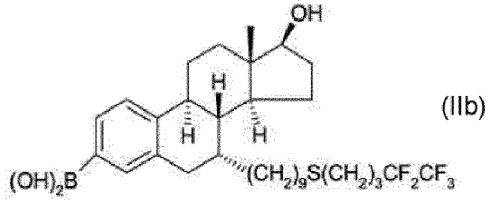 und
- Oxidationsreaktion der Verbindung der Formel (IIb) zum Bilden von Fulvestrant-3-Boronsäure (I).

7. Verbindung der Formel (III) nach Anspruch 1.

8. Verbindung nach Anspruch 7, die im XRPD-Diffraktogramm mindestens drei der folgenden charakterisierenden Peaks aufweist: 5,09, 8,03, 8,61, 10,20, 15,37, 17,72 ± 0,2 Grad 2θ.

## Revendications

1. Procédé de préparation d'acide fulvestrant 3-boronique (I) comprenant la transformation d'un composé de formule (IV) 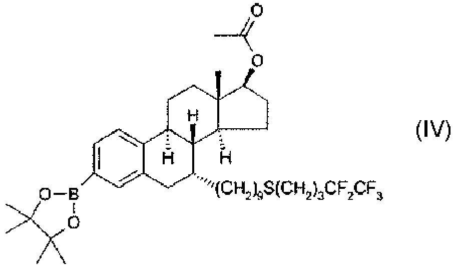 en un composé de formule (III)

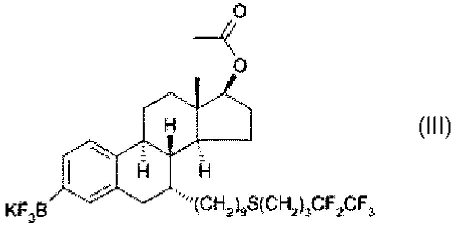

2. Procédé selon la revendication 1, ladite transformation se produisant par la réaction du composé de formule (IV) avec de l'hydrogénodifluorure de potassium dans un solvant de réaction approprié.

3. Procédé selon la revendication 2, ledit solvant étant un solvant miscible à l'eau choisi de préférence entre l'acétonitrile, le tétrahydrofurane, le méthanol, l'acétone, l'eau et leurs mélanges.

4. Procédé selon la revendication 3, ledit solvant étant un mélange d'eau et d'acétone.

5. Procédé selon la revendication 1 comprenant en outre les étapes suivantes :
- la réaction d'oxydation du composé de formule (III) pour donner un composé de formule (IIa) 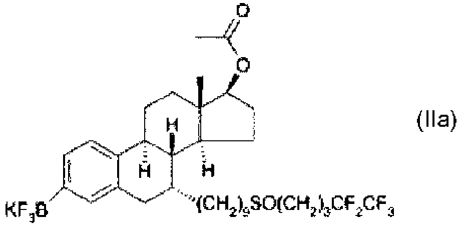 et
- la réaction d'hydrolyse du composé de formule (IIa) pour donner de l'acide fulvestrant 3-boronique (I).

6. Procédé selon la revendication 1 comprenant en outre les étapes suivantes :
- la réaction d'hydrolyse du composé de formule (III) pour donner le composé de formule (IIb) 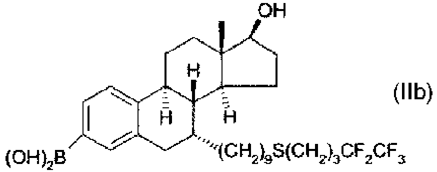 et
- la réaction d'oxydation du composé de formule (IIb) pour donner de l'acide fulvestrant 3-boronique (I).

7. Composé de formule (III), comme défini dans la revendication 1.

8. Composé selon la revendication 7 présentant dans le diffractogramme XRPD au moins trois des pics caractéristiques suivants : 5,09, 8,03, 8,61, 10,20, 15,37, 17,72 ± 0,2 degrés 2θ.
